(19) Europäisches Patentamt
European Patent Office
Office européen des brevets

(11) EP 2 146 582 B1

(12) **EUROPEAN PATENT SPECIFICATION**

(45) Date of publication and mention of the grant of the patent:
**15.03.2017 Bulletin 2017/11**

(21) Application number: **08759499.0**

(22) Date of filing: **09.05.2008**

(51) Int Cl.:
***A23C 9/12*** (2006.01) ***A23C 9/127*** (2006.01)
***C12N 9/80*** (2006.01)

(86) International application number:
**PCT/EP2008/055772**

(87) International publication number:
**WO 2008/138900 (20.11.2008 Gazette 2008/47)**

(54) **METHOD FOR PRODUCING AN ACIDIFIED MILK DRINK**

VERFAHREN ZUR HERSTELLUNG EINES GESÄUERTEN MILCHGETRÄNKS

MÉTHODE DE PRODUCTION D'UNE BOISSON LACTÉE ACIDULÉE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **11.05.2007 EP 07108070**

(43) Date of publication of application:
**27.01.2010 Bulletin 2010/04**

(73) Proprietor: **Chr. Hansen A/S**
**2970 Hoersholm (DK)**

(72) Inventors:
- **TAMS, Jeppe, Wegener**
  **DK-2820 Gentofe (DK)**
- **NIELSEN, Preben**
  **DK-2970 Hoersholm (DK)**

(56) References cited:
**EP-A1- 1 371 734 EP-A1- 1 839 491**
**EP-A2- 0 976 829 JP-A- 2003 250 460**

- **KAILASAPATHY ET AL: "Survival of free and encapsulated probiotic bacteria and their effect on the sensory properties of yoghurt" LEBENSMITTEL WISSENSCHAFT UND TECHNOLOGIE, ACADEMIC PRESS, LONDON, GB, vol. 39, no. 10, 1 December 2006 (2006-12-01), pages 1221-1227, XP005502593 ISSN: 0023-6438**
- **HARTE ET AL.: "Low-Fat Set Yogurt Made from Milk Subjected to Combinations of High Hydrostatic Pressure and Thermal Processing" JOURNAL OF DAIRY SCIENCE, vol. 4, no. 86, 2003, pages 1074-1082, XP002550300**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 146 582 B1

SEQUENCE LISTING

<110> Novozymes A/S

<120> Method for producing an acidified milk drink

<130> 11211.204-WO

<160> 5

<170> PatentIn version 3.4

<210> 1

<211> 957
<212> DNA
<213> Chryseobacterium

<400> 1

```
atgaaaaatc ttttttttatc aatgatggca tttgtaacaa ttttgtcatc tactgcctgc      60
tcggattcta gtgccaatca ggatcctaat ttaaccagta aagaagctaa tatcgctttg     120
aaagacctcg gaaagactgt tccggtaggg atagacgatg aaaatggaaa gctgaaagtt     180
tcatttattg tgacggccca gttttatact attgaatcca ccaaggaaaa tcaaaagtac     240
atcgacatca tcagacaggc tgtaaaagat gaatcacctg tgcatgttta cattatccct     300
aacacaacta ccatcgcgaa ggttgaaaaa ggaactgagg aagatgtggc ctatttcaaa     360
tctgcattaa ctaaggaagt acgcacagaa accagcaaat taaccagtgt tattcctaat     420
ctggctactt taaacagtct gtttgcgcag atcaaaagtc agtcttgcgg aactaccacg     480
gcttcatcac cttgcattac attcagatat cctgtagacg gatgttatgc aagagctcac     540
aaaatgagac agattttaat caacaacgga tatgacagcg aaaagcagtt tgtatacgga     600
aatctaaaag catctaccgg tacttgttgt gtagcatgga gttatcatgt agcgctattg     660
gttagcttta aaaattctgc cggagttact gaaaaaagaa ttattgaccc atccctttttt     720
tccagcggac ctgtaacgga tactgcctgg agaaatgcat gcatcaacac atcttgtggc     780
tcagcatccg tatcttctta tgcaaacact gctggaaatg tatattacag aagtccggcc     840
ggttctttac tgtatgacaa taattatatc aatacaaatt gtgtattgac cacattttca     900
ttattgtcag gatgttctcc ttcaccggct cctgatgtat ccagctgtgg attttaa        957
```

<210> 2
<211> 318
<212> PRT
<213> Chryseobacterium

<400> 2

```
Met Lys Asn Leu Phe Leu Ser Met Met Ala Phe Val Thr Ile Leu Ser
1               5                   10                  15
```

Ser Thr Ala Cys Ser Asp Ser Ser Ala Asn Gln Asp Pro Asn Leu Thr
            20                  25                  30

Ser Lys Glu Ala Asn Ile Ala Leu Lys Asp Leu Gly Lys Thr Val Pro
        35                  40                  45

Val Gly Ile Asp Asp Glu Asn Gly Lys Leu Lys Val Ser Phe Ile Val
    50                  55                  60

Thr Ala Gln Phe Tyr Thr Ile Glu Ser Thr Lys Glu Asn Gln Lys Tyr
65                  70                  75                  80

Ile Asp Ile Ile Arg Gln Ala Val Lys Asp Glu Ser Pro Val His Val
                85                  90                  95

Tyr Ile Ile Pro Asn Thr Thr Thr Ile Ala Lys Val Glu Lys Gly Thr
            100                 105                 110

Glu Glu Asp Val Ala Tyr Phe Lys Ser Ala Leu Thr Lys Glu Val Arg
        115                 120                 125

Thr Glu Thr Ser Lys Leu Thr Ser Val Ile Pro Asn Leu Ala Thr Leu
    130                 135                 140

Asn Ser Leu Phe Ala Gln Ile Lys Ser Gln Ser Cys Gly Thr Thr Thr
145                 150                 155                 160

Ala Ser Ser Pro Cys Ile Thr Phe Arg Tyr Pro Val Asp Gly Cys Tyr
                165                 170                 175

Ala Arg Ala His Lys Met Arg Gln Ile Leu Ile Asn Asn Gly Tyr Asp
            180                 185                 190

Ser Glu Lys Gln Phe Val Tyr Gly Asn Leu Lys Ala Ser Thr Gly Thr
        195                 200                 205

Cys Cys Val Ala Trp Ser Tyr His Val Ala Leu Leu Val Ser Phe Lys
    210                 215                 220

Asn Ser Ala Gly Val Thr Glu Lys Arg Ile Ile Asp Pro Ser Leu Phe
225                 230                 235                 240

Ser Ser Gly Pro Val Thr Asp Thr Ala Trp Arg Asn Ala Cys Ile Asn
                245                 250                 255

```
Thr Ser Cys Gly Ser Ala Ser Val Ser Ser Tyr Ala Asn Thr Ala Gly
            260                 265                 270

Asn Val Tyr Tyr Arg Ser Pro Ala Gly Ser Leu Leu Tyr Asp Asn Asn
        275                 280                 285

Tyr Ile Asn Thr Asn Cys Val Leu Thr Thr Phe Ser Leu Leu Ser Gly
    290                 295                 300

Cys Ser Pro Ser Pro Ala Pro Asp Val Ser Ser Cys Gly Phe
305                 310                 315
```

<210> 3
<211> 12
<212> PRT
<213> Chryseobacterium

<400> 3

```
Lys Leu Thr Ser Val Ile Pro Asn Leu Ala Thr Leu
1               5                   10
```

<210> 4
<211> 186
<212> PRT
<213> Chryseobacterium

<400> 4

```
Lys Leu Thr Ser Val Ile Pro Asn Leu Ala Thr Leu Asn Ser Leu Phe
1               5                   10                  15

Ala Gln Ile Lys Ser Gln Ser Cys Gly Thr Thr Thr Ala Ser Ser Pro
            20                  25                  30

Cys Ile Thr Phe Arg Tyr Pro Val Asp Gly Cys Tyr Ala Arg Ala His
        35                  40                  45

Lys Met Arg Gln Ile Leu Ile Asn Asn Gly Tyr Asp Ser Glu Lys Gln
    50                  55                  60

Phe Val Tyr Gly Asn Leu Lys Ala Ser Thr Gly Thr Cys Cys Val Ala
65                  70                  75                  80

Trp Ser Tyr His Val Ala Leu Leu Val Ser Phe Lys Asn Ser Ala Gly
                85                  90                  95

Val Thr Glu Lys Arg Ile Ile Asp Pro Ser Leu Phe Ser Ser Gly Pro
            100                 105                 110

Val Thr Asp Thr Ala Trp Arg Asn Ala Cys Ile Asn Thr Ser Cys Gly
        115                 120                 125

Ser Ala Ser Val Ser Ser Tyr Ala Asn Thr Ala Gly Asn Val Tyr Tyr
    130                 135                 140

Arg Ser Pro Ala Gly Ser Leu Leu Tyr Asp Asn Asn Tyr Ile Asn Thr
145                 150                 155                 160

Asn Cys Val Leu Thr Thr Phe Ser Leu Leu Ser Gly Cys Ser Pro Ser
                165                 170                 175

Pro Ala Pro Asp Val Ser Ser Cys Gly Phe
            180                 185
```

<210> 5
<211> 897
<212> DNA
<213> Chryseobacterium

<400> 5

```
tcggattcta gtgccaatca ggatcctaat ttaaccagta aagaagctaa tatcgctttg     60
aaagacctcg gaaagactgt tccggtaggg atagacgatg aaaatggaaa gctgaaagtt    120
tcatttattg tgacggccca gttttatact attgaatcca ccaaggaaaa tcaaaagtac    180
atcgacatca tcagacaggc tgtaaaagat gaatcacctg tgcatgttta cattatccct    240
aacacaacta ccatcgcgaa ggttgaaaaa ggaactgagg aagatgtggc ctatttcaaa    300
tctgcattaa ctaaggaagt acgcacagaa accagcaaat taaccagtgt tattcctaat    360
ctggctactt taaacagtct gtttgcgcag atcaaaagtc agtcttgcgg aactaccacg    420
gcttcatcac cttgcattac attcagatat cctgtagacg gatgttatgc aagagctcac    480
aaaatgagac agattttaat caacaacgga tatgacagcg aaaagcagtt tgtatacgga    540
aatctaaaag catctaccgg tacttgttgt gtagcatgga gttatcatgt agcgctattg    600
gttagcttta aaaattctgc cggagttact gaaaaaagaa ttattgaccc atccctttt    660
tccagcggac ctgtaacgga tactgcctgg agaaatgcat gcatcaacac atcttgtggc    720
tcagcatccg tatcttctta tgcaaacact gctggaaatg tatattacag aagtccggcc    780
ggttctttac tgtatgacaa taattatatc aatacaaatt gtgtattgac cacattttca    840
ttattgtcag gatgttctcc ttcaccggct cctgatgtat ccagctgtgg attttaa       897
```

## Description

### REFERENCE TO A SEQUENCE LISTING

This application contains a Sequence Listing in computer readable form. The computer readable form is incorporated herein by reference.

### REFERENCE TO A DEPOSIT OF BIOLOGICAL MATERIAL

This application contains a reference to a deposit of biological material, which deposit is incorporated herein by reference. For complete information see last page of the description.

### TECHNICAL FIELD

The present invention relates to a method for producing an acidified milk drink using an enzyme which reduces the isoelectric point of the milk proteins. The invention also relates to a novel enzyme having deamidase activity and its use in production of an acidified milk drink.

### BACKGROUND OF THE INVENTION

The market for acidified milk drinks, which includes fermented milk drinks and liquid yoghurt, is increasing worldwide and there is an interest in improving the quality and economics of this product.

Acidified milk drinks are generally produced by mixing acidified milk with a sugar syrup solution, and subjecting the mixture to a homogenization treatment. Acidification may take place through addition of a chemical, such as glucono delta-lactone (GDL), or it may be caused by fermentation of the milk with lactic acid bacteria. When such products are stored, however, casein, an ingredient of milk, often precipitates or associates and aggregates, and as a result the drinks tend to separate so that liquid whey collects on the surface. This process, which is often referred to as syneresis, decreases the quality of the acidified milk drinks.

Pectin, starch, modified starch, CMC, etc., are often used as stabilizers in acidified milk drinks, but due to the relatively high cost of such stabilizers, there is an interest in finding other and perhaps even better solutions. It is therefore an object of the present invention to provide a method for manufacturing of a stable acidified milk drink where the separation into curd and whey upon storage is reduced. The aim is to provide an alternative method for stabilization to either complement or partly replace the use of the stabilizers used in the art today.

Harte et al. (2003), J. Dairy Sci., 86: 1074-1082, discloses combined use of thermal treatment and high hydrostatic pressure during yoghurt production to reduce syneresis and thus allow for manufacturing of yoghurt with less stabilizers.

Enzymes which are able to reduce the isoelectric point of proteins in a manner that does not alter the amino acid chain length of the protein are known in the prior art. For instance, deamidating enzymes which increase the negative charge of proteins by acting on the amide groups to generate carboxyl groups have been found (EP976829, US6756221). Deamidation of milk proteins by such enzymes has been described (WO2006075772, JP2003250460, WO2002068671). These publications teach that proteins, e.g. milk proteins, can be deamidated to improve a range of properties when the modified proteins are used in food items, e.g. dairy products containing such modified proteins are reported to have better taste and texture. However, these publications are silent with respect to the use of protein modifying enzymes in the production of acidified milk drinks.

### SUMMARY OF THE INVENTION

The present inventors have surprisingly found that syneresis of acidified milk drinks can be decreased by adding an enzyme during the manufacture of the product which reduces the isoelectric point of the casein in the milk.

Consequently, the present invention relates to a method for producing an acidified milk drink, said method comprising:

a) acidifying a milk substrate comprising milk protein; and
b) treating with an enzyme having a protein modifying activity which reduces the isoelectric point of the milk protein;

wherein step b) is performed before, during or after step a).

Furthermore, the inventors have identified a novel enzyme having deamidase activity which is applicable in the method of the invention.

In another aspect, the invention therefore relates to an isolated polypeptide having deamidase activity, selected from the group consisting of:

a) a polypeptide having an amino acid sequence which is at least 91% identical to (i) SEQ ID NO: 2, or (ii) amino acids 133-318 thereof;
b) a polypeptide which is encoded by a polynucleotide which is at least 90% identical to (i) SEQ ID NO:1, or (ii) nucleotides 397-954 thereof;
c) a polypeptide which is encoded by a polynucleotide whose complement hybridizes under high stringency conditions with nucleotides 397-954 of SEQ ID NO: 1; and
d) a polypeptide which is encoded by the plasmid contained in *E. coli* DSM 19445.

## BRIEF DESCRIPTION OF THE FIGURE

Figure 1 shows a coomassie stained IEF gel with deamidase treated casein. The following samples were loaded on the IEF gel (from left): Lane 1: IEF marker; Lane 2: casein treated with supernatant of the deamidase clone; Lane 3: casein treated with supernatant prepared for loading to preparative IEF (this sample is about 4 times diluted compared to lane 2); Lanes 4-11: casein treated with pools of fractions from preparative IEF (pool 4 (lane 7) contains the deamidase enzyme); Lane 12 is the casein standard.

## DETAILED DISCLOSURE OF THE INVENTION

<u>Acidified milk drinks</u>

"Acidified milk drinks" according to the present invention include any drinkable products based on acidified milk substrates which can be produced according to the method of the invention. Acidification may take place because of addition of a chemical, such as lactic acid, citric acid or glucono delta-lactone (GDL), or it may be as a fermentation with a microorganism. Acidified milk drinks thus include fermented milk drinks and liquid yoghurt drinks.
Acidified milk drinks according to the invention are drinkable in the sense that they are in liquid form and consumed as beverages, i.e. they are suitable for drinking instead of being eaten with a spoon. "In liquid form" means that the products are in the fluid state of matter thus exhibiting a characteristic readiness to flow. Thus, the shape of a liquid is usually determined by the container it fills, in contrary to, e.g., a gel-like substance, which is soft but not free flowing, such as e.g. yoghurt or pudding.
An acidified milk drink according to the present invention may have a viscosity which is lower than 40 Pa (obtained at shear rate 300 pr. s), preferable less than 30 Pa, more preferably less than 20 Pa, and even more preferably between 15 and 20 Pa at a shear rate of 300 pr. s.
Viscosity of acidified milk drinks may be determined as follows:

Principle: This method is based on characterisation of texture by a viscometry measurement (constant rate). By a constant rate measurement, viscosity and shear rate are registered as function of shear rate. Selected and calculated parameters from the flow curves are extracted.
Materials: StressTech rheometer with CC 25 (bop / cup) measurement system.
Procedure: Before the measurements are started, the samples must be tempered to the right temperature at 13°C.

A flow curve is registered by increasing the deformation (shear rate: 0.2707 to 300 $s^{-1}$) folllowed by a decreasing of deformation (shear rate : 300 to 0,2707 $s^{-1}$).

Settings:

Normal force:

- Method: To Gab
- Max loading force: 10N
- Start measurement when normal force is below: 10N
- Time out: 1000sec.

- Approx. sample height: 1.000mm

Shear rate:

- 21 steps - up and down: 0.2707 - 0.3304 - 0.4923 - 0.7334 - 1 - 2 - 4 - 6 - 10 - 25 - 50 - 75 - 100 - 125 - 150 - 175 - 200 - 225 - 250 - 275 - 300.
- Delay time: 5sec.
- Integration time: 10sec.

Temperature and time:

- Equilibrium time: -
- Manual: 1 repetition
- Manual control: 13°C
- Temp. after measurement: 13°C

Advanced position:

- Position resolution: 85 y rad
- Approach to steady state, interval+-: 0,010
- Strength: 60%
- Reference curve: off

An acidified milk drink according to the present invention may have a pH of less than 4.6, preferably less than 4.4, more preferably less than 4.2 and even more preferably about pH 4 or less. In one aspect, the acidified milk drink has a pH of less than 3.8, such as less than 3.6.

An acidified milk drink according to the invention may have a fat content of 0 to 2%, preferably below 1.5%, below 1%, below 0.5% or of about 0.1% or less. The acidified milk drink may have a milk solid non-fat content of less than 20%, preferably less than 8.5%, less than 8%, less than 7.5%, less than 7%, less than 6.5%, less than 6%, or of about 5%.

An acidified milk drink according to the invention may have a milk protein content of less than 8%, preferably less than 4%, less than 3.5% or less than 3%. In one preferred aspect, the acidified milk drink has a milk protein content of about 2.5 to about 3%. In another preferred aspect, the acidified milk drink has a milk protein content of less than 2%, preferably a milk protein content of about 1%.

An acidified milk drink according to the invention may have a shelf life of more than 7 days, preferably more than 14 days, more preferably more than 28 days, such as more than 3 months.

Preferably, the acidified milk drink according to the present invention has an increased stability. The stability may be determined after having stored the acidified milk drink for an appropriate number of days by measuring the height of the whey collecting on the surface because of syneresis. It may also be determined after accelerated syneresis, such as by centrifugation.

<u>Method of producing an acidified milk drink</u>

As mentioned above, the method for producing an acidified milk drink according to the present invention comprises:

a) acidifying a milk substrate comprising milk protein; and
b) treating with an enzyme having a protein modifying activity which reduces the isoelectric point of the milk protein;

wherein step b) is performed before, during or after step a).

"Milk substrate", in the context of the present invention, may be any raw and/or processed milk material that can be subjected to acidification according to the method of the invention. Thus, useful milk substrates include, but are not limited to solutions/suspensions of any milk or milk like products comprising milk protein, such as whole or low fat milk, skim milk, buttermilk, reconstituted milk powder, condensed milk, dried milk, whey, whey permeate, lactose, mother liquid from crystallization of lactose, whey protein concentrate, or cream. Obviously, the milk substrate may originate from any mammal.

Preferably, the milk substrate is a lactose solution/suspension and more preferably the substrate is milk. In a preferred embodiment, the milk substrate is an aqueous solution of skim milk powder.

The milk substrate comprises milk protein, e.g. casein and whey protein. "Milk protein" in the context of the present invention may be any protein naturally occurring in milk. When referring to the milk protein in the acidified milk drink, the milk protein includes the enzymatically modified milk protein.

The term "milk" is to be understood as the lacteal secretion obtained by milking any mammal, such as cows, sheep, goats, buffaloes or camels.

In one embodiment, the milk substrate may be more concentrated than raw milk and it may thus have a protein content of more than 5%, preferably more than 6%, more than 7%, more than 8%, more than 9%, or more than 10%, and a lactose content of more than 7%, preferably more than 8%, more than 9%, or more than 10%.

Prior to acidification, the milk substrate may be homogenized and pasteurized according to methods known in the art.

"Homogenizing" as used herein means intensive mixing to obtain a soluble suspension or emulsion. If homogenization is performed prior to acidification, it may be performed so as to break up the milk fat into smaller sizes so that it no longer separates from the milk. This may be accomplished by forcing the milk at high pressure through small orifices.

"Pasteurizing" as used herein means heating to and maintaining at a specified high temperature for a specified period of time. Such high temperature may be, e.g., at least 65°C, at least 70°C, at least 75°C, at least 80°C, at least 85°C, at least 90°C or at least 95°C, and the milk substrate may be maintained at such high temperature for, e.g., at least 15 sec, at least 20 sec, at least 30 sec, at least 5 minutes or at least 10 minutes. Such pasteurization will reduce or eliminate the presence of live organisms, such as microorganisms, in the milk substrate. Pasteurization of the milk substrate may also denature wholly or partly some of the milk protein. If performed after the enzyme treatment, pasteurization may also inactivate the enzyme. A rapid cooling step may follow.

In the method of the present invention, the milk substrate is acidified. Such acidification may be a chemical acidification, such as by the addition of an acid, such as lactic acid or citric acid, or glucono delta-lactone (GDL), which is a cyclic ester of D-gluconic acid commonly used for acidification of food.

In a preferred aspect of the method of the invention, the acidification comprises fermentation of the milk substrate with a microorganism.

"Fermentation" in the method of the present invention means the conversion of carbohydrates into alcohols or acids through the action of a microorganism. Preferably, fermentation in the method of the present invention comprises conversion of lactose to lactic acid.

In the context of the present invention, "microorganism" may include any bacterium or fungus being able to ferment the milk substrate.

The microorganisms used for most fermented milk products are selected from the group of bacteria generally referred to as lactic acid bacteria. As used herein the term "lactic acid bacterium" designates a gram-positive, microaerophilic or anaerobic bacterium, which ferments sugars with the production of acids including lactic acid as the predominantly produced acid, acetic acid and propionic acid. The industrially most useful lactic acid bacteria are found within the order "Lactobacillales" which includes *Lactococcus* spp., *Streptococcus* spp., *Lactobacillus* spp., *Leuconostoc* spp., *Pseudoleuconostoc* spp., *Pediococcus* spp., *Brevibacterium* spp., *Enterococcus* spp. and *Propionibacterium* spp. Additionally, lactic acid producing bacteria belonging to the group of the strict anaerobic bacteria, bifidobacteria, i.e. *Bifidobacterium* spp., which are frequently used as food cultures alone or in combination with lactic acid bacteria, are generally included in the group of lactic acid bacteria.

Lactic acid bacteria are normally supplied to the dairy industry either as frozen or freeze-dried cultures for bulk starter propagation or as so-called "Direct Vat Set" (DVS) cultures, intended for direct inoculation into a fermentation vessel or vat for the production of a dairy product, such as an acidified milk drink. Such cultures are in general referred to as "starter cultures" or "starters".

Commonly used starter culture strains of lactic acid bacteria are generally divided into mesophilic organisms having optimum growth temperatures at about 30°C and thermophilic organisms having optimum growth temperatures in the range of about 40 to about 45°C. Typical organisms belonging to the mesophilic group include *Lactococcus lactis, Lactococcus lactis* subsp. *cremoris, Leuconostoc mesenteroides* subsp. *cremoris, Pseudoleuconostoc mesenteroides* subsp. *cremoris, Pediococcus pentosaceus, Lactococcus lactis* subsp. *lactis biovar. diacetylactis, Lactobacillus casei* subsp. *casei* and *Lactobacillus paracasei* subsp. *paracasei.* Thermophilic lactic acid bacterial species include as examples *Streptococcus thermophilus, Enterococcus faecium, Lactobacillus delbrueckii* subsp. *lactis, Lactobacillus helveticus, Lactobacillus delbrueckii* subsp. *bulgaricus* and *Lactobacillus acidophilus.*

Also the strict anaerobic bacteria belonging to the genus *Bifidobacterium* including *Bifidobacterium bifidum* and *Bifidobacterium longum* are commonly used as dairy starter cultures and are generally included in the group of lactic acid bacteria. Additionally, species of *Propionibacteria* are used as dairy starter cultures, in particular in the manufacture of cheese. Additionally, organisms belonging to the *Brevibacterium* genus are commonly used as food starter cultures.

Another group of microbial starter cultures are fungal cultures, including yeast cultures and cultures of filamentous fungi, which are particularly used in the manufacture of certain types of cheese and beverage. Examples of fungi include *Penicillium roqueforti, Penicillium candidum, Geotrichum candidum, Torula kefir, Saccharomyces kefir and Sac-*

*charomyces cerevisiae.*

In a preferred embodiment of the present invention, the microorganism used for fermentation of the milk substrate is *Lactobacillus casei* or a mixture of *Streptococcus thermophilus* and *Lactobacillus delbrueckii* subsp. *bulgaricus.*

Fermentation processes to be used in production of acidified milk drinks are well known and the person of skill in the art will know how to select suitable process conditions, such as temperature, oxygen, amount and characteristics of microorganism/s, additives such as e.g. carbohydrates, flavours, minerals, enzymes (e.g. rennet and phospholipase) and process time. Obviously, fermentation conditions are selected so as to support the achievement of the present invention, i.e. to obtain a fermented milk product suitable in the production of an acidified milk drink.

In one embodiment of the method of the present invention, a syrup is added to the milk substrate, either before or after acidification.

In a preferred embodiment, a syrup is added to the milk substrate after acidification, such as after fermentation.

"Syrup" in the context of the present invention is any additional additive ingredient giving flavour and/or sweetness to the final product, i.e. the acidified milk drink. It may be a solution comprising, e.g., sugar, sucrose, glucose, liquid sugar of fructose, aspartame, sugar alcohol, fruit concentrate, orange juice, strawberry juice and/or lemon juice.

The mixture of the acidified milk substrate and the syrup may be homogenized using any method known in the art. The homogenization may be performed so as to obtain a liquid homogenous solution which is smooth and stable. Homogenization of the mixture of the acidified milk substrate and the syrup may be performed by any method known in the art, such as by forcing the milk at high pressure through small orifices.

In one embodiment of the invention, water is added to the acidified milk substrate, such as to the fermented milk substrate, and the mixture of acidified milk substrate, such as fermented milk substrate, and water is homogenized.

In another embodiment of the method of the invention, the acidification of the milk substrate takes place because of addition of the syrup, which may be acidic, i.e. by mixing the milk substrate with syrup in the form of, e.g., fruit concentrate, orange juice, strawberry juice and/or lemon juice.

Step b) of the method of the present invention comprises an enzyme treatment. The enzyme treatment may be performed prior to step a), i.e. the milk substrate may be subjected to enzyme treatment before acidification, such as before addition of the chemical acidifier or before inoculation with the microorganism. The enzyme treatment may be performed during step a), i.e. the milk substrate may be subjected to enzyme treatment at the same time as it is being acidified. In one embodiment, the enzyme is added before or after inoculation of the milk substrate with a microorganism, and the enzyme reaction on the milk substrate takes place at the same time as it is being fermented.

Alternatively, the enzyme treatment may be performed after step a), i.e. the milk substrate may be subjected to enzyme treatment after acidification. If the acidified milk substrate is mixed and optionally homogenized with the syrup, the enzyme treatment may be performed before or after this. The enzyme may be added at the same time or after the syrup, but before homogenization, or it may be added after the acidified milk substrate and the syrup have been mixed and homogenized.

In a preferred embodiment, step b) is performed before or during step a). In a more preferred embodiment, the milk substrate is subjected to pasteurization prior to step a), and the enzyme treatment is performed after pasteurization but before or during acidification, e.g., the enzyme may be added before or after inoculation with the microorganism, and the enzyme reaction may take place at essentially the same time as the fermentation.

In an even more preferred embodiment, the milk substrate is subjected to pasteurization prior to step a), and the enzyme treatment is performed after pasteurization but before acidification. In an even more preferred embodiment, another pasteurization is performed after the enzyme treatment but before acidification, i.e., pasteurization is performed before and after step b), and step a) is performed after the second pasteurization.

The protein modifying enzyme is added in a suitable amount to achieve the desired degree of protein modification under the chosen reaction conditions. The enzyme may be added at a concentration of between 0.0001 and 1 g/L milk substrate, preferably between 0.01 and 0.1 g/L milk substrate.

The enzymatic treatment in the process of the invention may be conducted by adding the enzyme to the milk substrate and allowing the enzyme reaction to take place at an appropriate holding-time at an appropriate temperature. The enzyme treatment may be carried out at conditions chosen to suit the selected protein modifying enzyme according to principles well known in the art. The treatment may also be conducted by contacting the milk substrate with an enzyme that has been immobilised.

The enzyme treatment may be conducted at any suitable pH, such as e.g., in the range 2-10, such as, at a pH of 4-9 or 5-7. It may be preferred to let the enzyme act at the natural pH of the milk substrate, or, if acidification is obtained because of fermentation, the enzyme may act at the natural pH of the milk substrate during the fermentation process, i.e. the pH will gradually decrease from the natural pH of the unfermented milk substrate to the pH of the fermented milk substrate.

The enzyme treatment may be conducted at any appropriate temperature, e.g. in the range 1-70°C, such as 2-60°C. If the milk substrate is acidified because of fermentation with a microorganism, the enzyme treatment may be conducted during the fermentation, e.g. at 35-45°C.

Optionally, after the enzyme has been allowed to act on the milk substrate, the enzyme protein may be removed, reduced, and/or inactivated by any method known in the art.

Optionally, other ingredients may be added to the acidified milk drink, such as colour; stabilizers, e.g. pectin, starch, modified starch, CMC, etc.; or polyunsaturated fatty acids, e.g. omega-3 fatty acids. Such ingredients may be added at any point during the production process, i.e. before or after acidification, before or after enzyme treatment, and before or after the optional addition of syrup.

Enzyme having a protein modifying activity

In the method of the present invention, an enzyme is used to reduce the isoelectric point of milk protein in acidified milk drinks, thus decreasing the syneresis upon storage.

It is to be understood that in the context of the method of the invention, "milk protein" means any milk protein, i.e. any protein naturally occurring in milk. Thus, in the method of the present invention, the isoelectric point of any milk protein is reduced. In other words, in the method of the present invention, an enzyme is used to reduce the isoelectric point of at least one milk protein, i.e. at least one of the proteins naturally occurring in milk.

Thus, the method for producing an acidified milk drink according to the present invention in one embodiment comprises:

a) acidifying a milk substrate comprising a milk protein; and
b) treating with an enzyme having a protein modifying activity which reduces the isoelectric point of the milk protein;

wherein step b) is performed before, during or after step a).

In a preferred embodiment, the milk protein is casein.

Preferentially, the enzyme to be used has an activity which reduces the isoelectric point of the protein in a manner that does not alter the amino acid chain length of the protein, i.e. without hydrolysing the peptide bonds in the protein and without adding new amino acids to the N-terminal or the C-terminal of the amino acid chain.

In one aspect of the present invention, the isoelectric point of the milk protein is reduced by introducing more negative charges in the milk protein at low pH. Such negative charges may be added on the surface of milk protein, e.g., casein.

In a preferred aspect of the method of the invention, the enzyme having a protein modifying activity which reduces the isoelectric point of the milk protein is an enzyme which introduces additional negative charges into the protein in a manner that does not alter the amino acid chain length of the protein. I.e., the additional negative charges are not introduced by, e.g., increasing the amino acid chain length by addition of more (negatively charged) amino acids at the C-terminal or the N-terminal of the amino acid chain. The additional negative charges are preferentially introduced by modifying amino acid side chains in the protein. In a preferred aspect, such modification does not comprise cross-linking of the protein. The enzyme may have an activity which deamidates amide groups in the protein by directly acting upon such groups without hydrolysing peptide bonds of the protein and without cross-linking of the protein. The enzyme may, e.g., be an asparaginase, a glutaminase, an amidase or a deamidase.

In a more preferred aspect, the enzyme has deamidase activity.

The term "deamidase" refers to an enzyme that catalyzes the hydrolysis of the gamma-amide of the amino acid glutamine or asparagine incorporated in a polypeptide chain (protein) and releases side chain carboxyl groups and ammonia. Such an enzyme may also be referred to as peptidoglutaminase, protein-deamidase, protein deamidating enzyme, protein glutamine glutaminase, protein glutamine amidohydrolase. The group of deamidases comprises but is not limited to the enzymes assigned to subclasses EC 3.5.1.44 (described in Kikuchi, M., Hayashida, H., Nakano, E. and Sakaguchi, K. Peptidoglutaminase. Enzymes for selective deamidation of y-amide of peptide-bound glutamine. Biochemistry 10 (1971) 1222-1229).

Enzymes having a protein modifying activity to be used in the method of the present invention may be of animal, of plant or of microbial origin. Preferred enzymes are obtained from microbial sources, in particular from a filamentous fungus or yeast, or from a bacteria.

The enzyme may, e.g., be derived from a strain of *Aspergillus,* e.g. *A. niger, A. awamori, A. foetidus, A. japonicus, A. oryzae; Dictyostelium,* e.g. *D. discoideum; Mucor,* e.g. *M. javanicus, M. mucedo, M. subtilissimus; Neurospora,* e.g. *N. crassa; Rhizomucor,* e.g. *R. pusillus; Rhizopus,* e.g. *R. arrhizus, R. japonicus, R. stolonifer; Sclerotinia,* e.g. *S. libertiana; Trichophyton,* e.g. *T. rubrum; Whetzelinia,* e.g. *W. sclerotiorum; Bacillus,* e.g. *B. megaterium, B. subtilis, B. pumilus, B. stearothermophilus; Chryseobacterium; Citrobacter,* e.g. *C. freundii; Enterobacter,* e.g. *E. aerogenes, E. cloacae Edwardsiella, E. tarda; Erwinia,* e.g. *E. herbicola; Escherichia,* e.g. *E. coli; Klebsiella,* e.g. *K. pneumoniae; Proteus,* e.g. *P. vulgaris; Providencia,* e.g. *P. stuartii; Salmonella,* e.g. *S. typhimurium; Serratia,* e.g. *S. liquefasciens, S. marcescens; Shigella,* e.g. *S. flexneri; Streptomyces,* e.g. *S. violeceoruber; Yersinia,* e.g. *Y. enterocolitica.*

In a preferred embodiment, the enzyme is bacterial, e.g. from the class Flavobacteria, such as from the order Flavobacteriales, such as from the family Flavobacteriaceae, such as from a strain of *Chryseobacterium.* A preferred enzyme is a deamidase having a sequence which is at least 50%, such as at least 60%, at least 70%, at least 80% or at least 90% identical to SEQ ID NO: 2 or SEQ ID NO: 4.

Novel enzyme having deamidase activity

The present inventors have identified a novel enzyme from a strain of *Chryseobacterium* having deamidase activity. The DNA sequence of the open reading frame of the gene encoding the enzyme is disclosed as SEQ ID NO: 1. The amino acid sequence of the protein encoded by the gene is shown as SEQ ID NO: 2. SEQ ID NO: 3 shows the N-terminal sequence of the active deamidase as has been experimentally determined. SEQ ID NO: 4 shows the 186 amino acids long amino acid sequence of the mature enzyme. The mature enzyme has a molecular weight of ~20 kDa, as has been determined by SDS-PAGE. SEQ ID NO: 5 shows the DNA sequence of the enzyme encoding gene without the region encoding the signal peptide.

Thus, one aspect of the present invention relates to a polypeptide having deamidase activity, selected from the group consisting of:

a) a polypeptide having an amino acid sequence which is at least 75%, preferably at least 80% or at least 90%, more preferably at least 91%, at least 95% or at least 97%, identical to (i) SEQ ID NO: 2, or (ii) amino acids 133-318 thereof;
b) a polypeptide which is encoded by a polynucleotide which is at least 80%, preferably at least 90%, more preferably at least 95% or at least 97%, identical to (i) SEQ ID NO:1, or (ii) nucleotides 397-954 thereof;
c) a polypeptide which is encoded by a polynucleotide whose complement hybridizes under high stringency, preferably very high stringency, conditions, with nucleotides 397-954 of SEQ ID NO: 1; and
d) a polypeptide which is encoded by the plasmid contained in *E. coli* DSM 19445.

*The polypeptide may be isolated.*

The term "isolated polypeptide" as used herein refers to a polypeptide which is at least 20% pure, preferably at least 40% pure, more preferably at least 60% pure, even more preferably at least 80% pure, most preferably at least 90% pure, and even most preferably at least 95% pure, as determined by SDS-PAGE.

In a preferred aspect, a polypeptide of the present invention has an amino acid sequence which differs by ten amino acids, preferably by five amino acids, more preferably by four amino acids, even more preferably by three amino acids, most preferably by two amino acids, and even most preferably by one amino acid from SEQ ID NO: 2 or amino acids 133 to 318 thereof.

In another preferred aspect, a polypeptide of the present invention comprises the amino acid sequence of SEQ ID NO: 2 or an allelic variant thereof; or a fragment thereof that has deamidase activity, the fragment comprising at least 100, such as at least 150 or 200 amino acids. In a more preferred aspect, the polypeptide comprises the amino acid sequence of SEQ ID NO: 2. In another preferred aspect, the polypeptide comprises amino acids 133 to 318 of SEQ ID NO: 2, or an allelic variant thereof, or a fragment thereof that has deamidase activity, the fragment comprising at least 100, such as at least 150 or 200 amino acids. In another preferred aspect, the polypeptide consists of the amino acid sequence of SEQ ID NO: 2 or an allelic variant thereof; or a fragment thereof that has deamidase activity, the fragment comprising at least 100, such as at least 150 or 200 amino acids. In another preferred aspect, the polypeptide consists of amino acids 133 to 318 of SEQ ID NO: 2, or an allelic variant thereof, or a fragment thereof that has deamidase activity, the fragment comprising at least 100, such as at least 150 or 200 amino acids.

An "allelic variant" of a polypeptide is a polypeptide encoded by an allelic variant of a gene. An allelic variant of a gene is any of two or more alternative forms of a gene occupying the same chromosomal locus. Allelic variation arises naturally through mutation, and may result in polymorphism within populations. Gene mutations can be silent (no change in the encoded polypeptide) or may encode polypeptides having altered amino acid sequences, i.e. allelic variants of the polypeptide.

*Polynucleotide encoding a deamidase*

Another aspect of the present invention relates to an isolated polynucleotide encoding a polypeptide having deamidase activity, selected from the group consisting of:

a) a polynucleotide encoding a polypeptide having an amino acid sequence which is at least 75%, preferably at least 80% or at least 90%, more preferably at least 91%, at least 95% or at least 97%, identical to (i) SEQ ID NO: 2, or (ii) amino acids 133 to 318 thereof;

b) a polynucleotide which is at least 80%, preferably at least 90%, more preferably at least 95% or at least 97%, identical to (i) SEQ ID NO:1, or (ii) nucleotides 397-954 thereof; and

c) a polynucleotide whose complement hybridizes under high stringency conditions, preferably very high stringency conditions, with (i) SEQ ID NO: 1, (ii) nucleotides 397 to 954 of SEQ ID NO: 1, or (iii) a subsequence of (i) or (ii).

The term "subsequence" is defined herein as a nucleotide sequence having one or more nucleotides deleted from the 5' and/or 3' end of SEQ ID NO: 1 or a homologous sequence thereof. A subsequence of SEQ ID NO: 1 preferably contains at least 100 contiguous nucleotides, more preferably at least 200, 300, 400 or 500 contiguous nucleotides. A preferred subsequence consists of nucleotides 397 to 954 of SEQ ID NO: 1. Moreover, the subsequence may encode a polypeptide fragment which has deamidase activity.

For purposes of the present invention, hybridization indicates that the nucleotide sequence or its complement hybridizes to a labelled nucleic acid probe corresponding to the nucleotide sequence shown in SEQ ID NO: 1, its complementary strand, or a subsequence of any of these, under high to very high stringency conditions. A preferred subsequence consists of nucleotides 397 to 954 of SEQ ID NO: 1 or its complementary strand. Molecules to which the nucleic acid probe hybridizes under these conditions can be detected using X-ray film.

In a preferred aspect, the nucleic acid probe is a polynucleotide sequence which encodes the polypeptide of SEQ ID NO: 2, or a subsequence thereof, such as amino acids 133 to 318, or the complementary strand of any of these. In another preferred aspect, the nucleic acid probe is SEQ ID NO: 1 or nucleotides 397 to 954 thereof, or the complement of any of these. In another preferred aspect, the nucleic acid probe is the mature polypeptide coding region of SEQ ID NO: 1 or its complement.

For long probes of at least 100 nucleotides in length, high to very high stringency conditions are defined as prehybridization and hybridization at 42°C in 5X SSPE, 0.3% SDS, 200 μg/ml sheared and denatured salmon sperm DNA, and 50% formamide, following standard Southern blotting procedures for 12 to 24 hours optimally (J. Sambrook, E.F. Fritsch, and T. Maniatis, 1989, Molecular Cloning, A Laboratory Manual, 2d edition, Cold Spring Harbor, New York).

For long probes of at least 100 nucleotides in length, the carrier material is finally washed three times each for 15 minutes using 2X SSC, 0.2% SDS preferably at least at 65°C (high stringency), or more preferably at least at 70°C (very high stringency).

In a particular embodiment, the wash is conducted using 0.2X SSC, 0.2% SDS preferably at least at 65°C (high stringency), or more preferably at least at 70°C (very high stringency). In another particular embodiment, the wash is conducted using 0.1X SSC, 0.2% SDS preferably at least at 65°C (high stringency), or more preferably at least at 70°C (very high stringency).

For short probes which are about 15 nucleotides to about 70 nucleotides in length, stringency conditions are defined as prehybridization, hybridization, and washing post-hybridization at about 5°C to about 10°C below the calculated $T_m$ using the calculation according to Bolton and McCarthy (1962, Proceedings of the National Academy of Sciences USA 48:1390) in 0.9 M NaCl, 0.09 M Tris-HCl pH 7.6, 6 mM EDTA, 0.5% NP-40, 1X Denhardt's solution, 1 mM sodium pyrophosphate, 1 mM sodium monobasic phosphate, 0.1 mM ATP, and 0.2 mg of yeast RNA per ml following standard Southern blotting procedures.

For short probes which are about 15 nucleotides to about 70 nucleotides in length, the carrier material is washed once in 6X SCC plus 0.1 % SDS for 15 minutes and twice each for 15 minutes using 6X SSC at 5°C to 10°C below the calculated $T_m$.

Under salt-containing hybridization conditions, the effective $T_m$ is what controls the degree of identity required between the probe and the filter bound DNA for successful hybridization.

The effective $T_m$ may be determined using the formula below to determine the degree of identity required for two DNAs to hybridize under various stringency conditions.

$$\text{Effective } T_m = 81.5 + 16.6(\log M[Na^+]) + 0.41(\%G+C) - 0.72(\% \text{ formamide})$$

(See www.ndsu.nodak.edu/instruct/mcclean/plsc731/dna/dna6.htm)

The G+C content of SEQ ID NO: 1 is 39.3%. For medium stringency, the formamide is 35% and the $Na^+$ concentration for 5X SSPE is 0.75 M. Applying this formula to these values, the Effective $T_m$ is 70.3°C.

Another relevant relationship is that a 1 % mismatch of two DNAs lowers the $T_m$ by 1.4°C. To determine the degree of identity required for two DNAs to hybridize under medium stringency conditions at 42°C, the following formula is used:

$$\% \text{ Homology} = 100 - [(\text{Effective } T_m - \text{Hybridization Temperature})/1.4]$$

(See www.ndsu.nodak.edu/instruct/mcclean/plsc731/dna/dna6.htm)

Applying this formula to the values, the degree of identity required for two DNAs to hybridize under medium stringency conditions at 42°C is 100 - [(70.3 - 42)/1.4] = 79.8%.

*Sequence Identity*

The relatedness between two amino acid sequences is described by the parameter "identity" or "homology". For purposes of the present invention, the alignment of two amino acid sequences is determined by using the Needle program from the EMBOSS package (Rice, P., Longden, I. and Bleasby, A. (2000) EMBOSS: The European Molecular Biology Open Software Suite. Trends in Genetics 16, (6) pp276-277; http://emboss.org) version 2.8.0. The Needle program implements the global alignment algorithm described in Needleman, S. B. and Wunsch, C. D. (1970) J. Mol. Biol. 48, 443-453. The substitution matrix used is BLOSUM62, gap opening penalty is 10, and gap extension penalty is 0.5.

The degree of identity or homology between an amino acid sequence of the present invention ("invention sequence") and a different amino acid sequence ("foreign sequence") is calculated as the number of exact matches in an alignment of the two sequences, divided by the length of the "invention sequence" or the length of the "foreign sequence", whichever is the shortest. The result is expressed in percent identity.

An exact match occurs when the "invention sequence" and the "foreign sequence" have identical amino acid residues in the same positions of the overlap. The length of a sequence is the number of amino acid residues in the sequence.

In a particular embodiment, the percentage of identity of an amino acid sequence of a polypeptide with, or to, SEQ ID NO: 2 is determined by i) aligning the two amino acid sequences using the Needle program, with the BLOSUM62 substitution matrix, a gap opening penalty of 10, and a gap extension penalty of 0.5; ii) counting the number of exact matches in the alignment; iii) dividing the number of exact matches by the length of the shortest of the two amino acid sequences, and iv) converting the result of the division of iii) into percentage. The percentage of identity to, or with, other sequences of the invention are calculated in an analogous way.

For purposes of the present invention, the degree of identity between two nucleotide sequences is determined by the Wilbur-Lipman method (Wilbur and Lipman, 1983, Proceedings of the National Academy of Science USA 80: 726-730) using the LASERGENE™ MEGALIGN™ software (DNASTAR, Inc., Madison, WI) with an identity table and the following multiple alignment parameters: Gap penalty of 10 and gap length penalty of 10. Pairwise alignment parameters are Ktuple=3, gap penalty=3, and windows=20.

*Expression vectors and host cells*

Yet another aspect of the invention relates to nucleic acid constructs, recombinant expression vectors, and recombinant host cells comprising the polynucleotides, and to methods for producing such polypeptides having deamidase activity comprising (a) cultivating a recombinant host cell comprising a nucleic acid construct comprising a polynucleotide encoding the polypeptide under conditions conducive for production of the polypeptide; and (b) recovering the polypeptide.

The term "nucleic acid construct" as used herein refers to a nucleic acid molecule, either single- or double-stranded, which is isolated from a naturally occurring gene or which is modified to contain segments of nucleic acids in a manner that would not otherwise exist in nature. The term nucleic acid construct is synonymous with the term "expression cassette" when the nucleic acid construct contains the control sequences required for expression of a coding sequence of the present invention.

The term "control sequences" is defined herein to include all components, which are necessary or advantageous for the expression of a polynucleotide encoding a polypeptide of the present invention. Each control sequence may be native or foreign to the nucleotide sequence encoding the polypeptide. Such control sequences include, but are not limited to, a leader, polyadenylation sequence, propeptide sequence, promoter, signal peptide sequence, and transcription terminator. At a minimum, the control sequences include a promoter, and transcriptional and translational stop signals. The control sequences may be provided with linkers for the purpose of introducing specific restriction sites facilitating ligation of the control sequences with the coding region of the nucleotide sequence encoding a polypeptide.

The term "coding sequence" means a nucleotide sequence, which directly specifies the amino acid sequence of its protein product. The boundaries of the coding sequence are generally determined by an open reading frame, which usually begins with the ATG start codon or alternative start codons such as GTG and TTG. The coding sequence may be a DNA, a cDNA, or a recombinant nucleotide sequence.

The term "expression" includes any step involved in the production of the polypeptide including, but not limited to, transcription, post-transcriptional modification, translation, post-translational modification, and secretion.

The term "expression vector" is defined herein as a linear or circular DNA molecule that comprises a polynucleotide encoding a polypeptide of the invention, and which is operably linked to additional nucleotides that provide for its expression.

The term "operably linked" denotes herein a configuration in which a control sequence is placed at an appropriate position relative to the coding sequence of the polynucleotide sequence such that the control sequence directs the expression of the coding sequence of a polypeptide.

The term "host cell", as used herein, includes any cell type which is susceptible to transformation, transfection, transduction, and the like with a nucleic acid construct comprising a polynucleotide of the present invention.

*Sources of Polypeptides Having Deamidase Activity*

The nucleotide sequence of SEQ ID NO: 1 or a subsequence thereof, as well as the amino acid sequence of SEQ ID NO: 2 or a fragment thereof, may be used to design a nucleic acid probe to identify and clone DNA encoding polypeptides having deamidase activity from strains of different genera or species according to methods well known in the art. In particular, such probes can be used for hybridization with the genomic or cDNA of the genus or species of interest, following standard Southern blotting procedures, in order to identify and isolate the corresponding gene therein. Such probes can be considerably shorter than the entire sequence, but should be at least 14, preferably at least 25, more preferably at least 35, and most preferably at least 70 or at least 100 nucleotides in length. For example, the nucleic acid probe may be at least 200 nucleotides, preferably at least 300 nucleotides, more preferably at least 400 nucleotides, or most preferably at least 500 nucleotides in length. Even longer probes may be used, e.g., nucleic acid probes which are at least 600 nucleotides, preferably at least 700 nucleotides, more preferably at least 800 nucleotides, or most preferably at least 900 nucleotides in length. Both DNA and RNA probes can be used. The probes are typically labeled for detecting the corresponding gene (for example, with $^{32}P$, $^{3}H$, $^{35}S$, biotin, or avidin). Such probes are encompassed by the present invention.

A genomic DNA or cDNA library prepared from other organisms of interest may be screened for DNA which hybridizes with the probes described above and which encodes a polypeptide having deamidase activity. Genomic or other DNA from such other organisms may be separated by agarose or polyacrylamide gel electrophoresis, or other separation techniques. DNA from the libraries or the separated DNA may be transferred to and immobilized on nitrocellulose or other suitable carrier material. In order to identify a clone or DNA which is homologous with SEQ ID NO: 1 or a subsequence thereof, the carrier material is used in a Southern blot.

A polypeptide of the present invention may be obtained from microorganisms of any genus. For purposes of the present invention, the term "obtained from" as used in connection with a given source shall mean that the polypeptide encoded by a nucleotide sequence is produced by the source or by a strain in which the nucleotide sequence from the source has been inserted. In a preferred aspect, the polypeptide obtained from a given source is secreted extracellularly.

A polypeptide of the present invention may be a bacterial polypeptide, and preferably it may be obtained from or obtainable from the class Flavobacteria, more preferably from the order Flavobacteriales and even more preferably from the family Flavobacteriaceae. The polypeptide may be obtained from or obtainable from any of the genera *Bergeyella, Capnocytophaga, Cellulophaga, Chryseobacterium, Coenonia, Dokdonia, Empedobacter, Flavobacterium, Gelidibacter, Ornithobacterium, Polaribacter, Psychroflexus, Psychroserpens, Riemerella, Saligentibacter,* or *Weeksella,* preferably from *Chryseobacterium.*

For the aforementioned species, the invention encompasses both the perfect and imperfect states, and other taxonomic equivalents, e.g., anamorphs, regardless of the species name by which they are known. Those skilled in the art will readily recognize the identity of appropriate equivalents.

Strains of the genera mentioned above are readily accessible to the public in a number of culture collections, such as the American Type Culture Collection (ATCC), Deutsche Sammlung von Mikroorganismen und Zellkulturen GmbH (DSMZ), Centraalbureau Voor Schimmelcultures (CBS), and Agricultural Research Service Patent Culture Collection, Northern Regional Research Center (NRRL).

Furthermore, polypeptides of the present invention may be identified and obtained from other sources including microorganisms isolated from nature (e.g., soil, composts, water, etc.) using the above-mentioned probes. Techniques for isolating microorganisms from natural habitats are well known in the art. The polynucleotide may then be obtained by similarly screening a genomic or cDNA library of another microorganism. Once a polynucleotide sequence encoding a polypeptide has been detected with the probe(s), the polynucleotide can be isolated or cloned by utilizing techniques which are well known to those of ordinary skill in the art (see, e.g., Sambrook et al., 1989, supra).

Polypeptides of the present invention also include fused polypeptides or cleavable fusion polypeptides in which another polypeptide is fused at the N-terminus or the C-terminus of the polypeptide or fragment thereof. A fused polypeptide is produced by fusing a nucleotide sequence (or a portion thereof) encoding another polypeptide to a nucleotide sequence (or a portion thereof) of the present invention. Techniques for producing fusion polypeptides are known in the art, and include ligating the coding sequences encoding the polypeptides so that they are in frame and that expression of the fused polypeptide is under control of the same promoter(s) and terminator.

## EXAMPLES

### Example 1

SKMP solution

20 ml water + 4.5 g skim milk powder (instant dispersibility from Kerry) was incubated at 50°C for 10 min before use, so a homogeneous solution was obtained.

Sugar solution

3.3 g sucrose
10.5 g glucose
These sugars were added to 46 ml 20 mM lactic acid buffer, pH 4.0 and incubated at 90°C for 5 min with stirring and then cooled down to 5°C.

Enzyme

Chromatographically purified *Chryseobacterium* deamidase (Example 3), 0.9 mg/ml, was diluted to give the final concentrations indicated in the Tables.

Procedure A (Enzyme added before pasteurization)

250 ul SKMP solution was transferred to eppendorf tubes. 20 ul Enzyme or water (control) was added and incubation was performed for 120 min at 50°C.
The solution was incubated at 85°C for 30 min with 1000 rpm and hereafter incubated at 43°C for 10 min with 1000 rpm.
30 ul 4 U/l YF-3331 (mixed strain culture containing *Streptococcus thermophilus* and *Lactobacillus delbrueckii* subsp. *bulgaricus* from Chr. Hansen A/S, Denmark) was added and incubation was performed for 16 hours at 43°C.
Hereafter the samples were incubated at 0-5°C ice/water bath for 20 min.
600 ul sugar solution (ice bath) was added and sucked up and down with a pipette five times.
500 ul glass beads (5°C) were added to each tube.
The samples were placed in an eppendorf thermomixer at 5°C with 1400 rpm for 10 min.
The samples were placed at 5°C for 4 days and syneresis was measured.

Procedure B (Enzyme added after pasteurization)

250 ul SKMP solution was transferred to eppendorf tubes and incubated for 120 min at 50°C.
The solution was incubated at 85°C for 30 min with 1000 rpm and hereafter incubated at 43°C for 10 min with 1000 rpm.
30 ul 4 U/l YF-3331 (mixed strain culture containing *Streptococcus thermophilus* and *Lactobacillus delbrueckii* subsp. *bulgaricus* from Chr. Hansen A/S, Denmark) + 20 ul Enzyme or water (control) was added and incubation was performed for 16 hours at 43°C.
Hereafter the samples were incubated at 0-5°C ice/water bath for 20 min.
600 ul sugar solution (ice bath) was added and sucked up and down with a pipette five times.
500 ul glass beads (5°C) were added to each tube.
The samples were placed in an eppendorf thermomixer at 5°C with 1400 rpm for 10 min.
The samples were placed at 5°C for 4 days and syneresis was measured.

Procedure C (Enzyme added after homogenization)

250 ul SKMP solution was transferred to eppendorf tubes and incubated for 120 min at 50°C.
The solution was incubated at 85°C for 30 min with 1000 rpm and hereafter incubated at 43°C for 10 min with 1000 rpm.
30 ul 4 U/l YF-3331 (mixed strain culture containing *Streptococcus thermophilus* and *Lactobacillus delbrueckii* subsp.

*bulgaricus* from Chr. Hansen A/S, Denmark) was added and incubation was performed for 16 hours at 43°C.
Hereafter the samples were incubated at 0-5°C ice/water bath for 20 min.
600 ul sugar solution (ice bath) was added + 20 ul Enzyme or water (control) and sucked up and down with a pipette five times.
500 ul glass beads (5°C) were added to each tube.
The samples were placed in an eppendorf thermomixer at 5°C with 1400 rpm for 10 min.
The samples were placed at 5°C for 4 days and syneresis was measured.

The data (double determinations) in Table 1 shows a decreased syneresis when deamidase is added compared to the water control. The most predominant effect can be seen when the enzyme is added after pasteurization.

| Table 1 | Deamidase ug/ml | Average syneresis mm | deviation (+/-) mm |
|---|---|---|---|
| **Procedure A** | | | |
| Added before pasteurization | 57 | 2.8 | 0.08 |
| Water control | 0 | 3.3 | 0.04 |
| | | | |
| **Procedure B** | | | |
| Added after pasteurization | 57 | 1.1 | 0.03 |
| Water control | 0 | 3.5 | 0.25 |
| **Procedure C** | | | |
| Added after homogenization | 57 | 3.9 | 0.12 |
| Water control | 0 | 3.8 | 0.13 |

## Example 2

A dose response experiment was made according to Procedure B as described in Example 1.

Table 2 shows a decrease in syneresis with increasing deamidase concentration.

| Table 2 | Deamidase ug/ml | Average syneresis Mm | deviation (+/-) mm |
|---|---|---|---|
| Added after pasteurization | 0 | 2.5 | 0.11 |
| Added after pasteurization | 13 | 2.2 | 0.10 |
| Added after pasteurization | 29 | 1.6 | 0.00 |
| Added after pasteurization | 57 | 1.2 | 0.03 |

## Example 3

Cloning and production of deamidase

Cloning and production

*Chryseobacterium* sp. was isolated from a soil sample from Antarctica from 19/01/1989.

The deamidase gene was PCR amplified based on primers designed from the sequences of known deamidases. (Genbank Acc no AB046594 (Eur J Biochem vol 268:1410-1421, 2001) and GenseqN Acc no AAZ49495 (EP976829)). The sequence obtained from such product is shown as SEQ ID NO: 1.

The signal peptide from the SavinaseTM gene (the subtilisin protease of *B .clausii*) was fused by PCR in frame to the gene encoding the deamidase (SEQ ID NO: 5).

The fused gene was integrated by homologous recombination into the genome of the *Bacillus subtilis* host. The gene construct was expressed under the control of a triple promoter system (as described in WO 99/43835), consisting of the promoters from *Bacillus licheniformis* alpha-amylase gene (*amyL*), *Bacillus amyloliquefaciens* alpha-amylase gene (*amyQ*), and *Bacillus thuringiensis cryIIIA* promoter including stabilizing sequence. The gene coding for Chloramphenicol acetyl-transferase was used as marker. (Described e.g in Diderichsen et al., A useful cloning vector for Bacillus subtilis. Plasmid, 30, p. 312, 1993). Chloramphenicol resistant transformants were analyzed by DNA sequencing to verify the correct DNA sequence of the construct. One such clone was selected.

Fermentations of the deamidase expression clone was performed on a rotary shaking table in 500 ml baffled Erlenmeyer flasks each containing 100 ml LB medium (or another medium suitable for fermentation of Bacillus subtilis) supplemented with 34 mg/l chloramphenicol. The clone was fermented for 5 days at 30°C.

Purification of deamidase by preparative IEF

50 ml of supernatant was added in 2.5 ml aliquots to PD10TM (Pharmacia) gelfiltration columns to remove salt. 70 ml was collected from the PD10 TM (Pharmacia) columns. Sample was prepared for RotoforTM (Biorad) separation. 59 ml gelfiltrated supernant was mixed with 3 ml ampholyte Biolyte 3/10 (Biorad art 163 1112). The preparative isoelectric focusing (IEF) was conducted as recommended by the manufacturer. Sample was loaded in the RotoforTM and electrophoresed for 4 hours with constant effect of 15W. Twenty fractions obtained by the preparative IEF were evaluated on SDS PAGE, and based on the banding patterns the fractions were pooled. The activity was evaluated on casein by a Qualitative assay as described below.

Qualitative deamidase assay

Casein was deamidated by adding 10 ul sample to 1 ml 1% casein in Britten Robinson buffer pH 7, followed by incubation at 37°C for 1 h with agitation. Reaction was stopped by taking the sample to ice.

The deamidation of casein was evaluated by analytical IEF. The enzyme treated samples (and an untreated casein control) were mixed 1:1 with a solution of 9 M Urea, 2% Triton X-100 to denature the casein micelles. Samples were loaded on Pharmacia IEF 3/10. The IEF gel was subjected to electrophoresis as recommended by the manufacturer, followed by coomassie staining to visualise the protein (Figure 1). The following samples were loaded on the IEF gel (from left) Lane 1: IEF marker; Lane 2: supernatant of the deamidase clone; Lane 3 is the supernatant prepared for loading to preparative IEF (this sample is about 4 times diluted compared to lane 2); Lanes 4-11 are pools of fractions from preparative IEF (pool 4 (lane 7) contains the deamidase enzyme); Lane 12 is the casein standard.

**DEPOSIT OF BIOLOGICAL MATERIAL**

The following biological material has been deposited under the terms of the Budapest Treaty with the Deutsche Sammlung von Mikroorganismen und Zellkulturen GmbH (DSM), Mascheroder Weg 1 B, D-38124 Braunschweig, Germany, and given the following accession number:

| Deposit | Accession Number | Date of Deposit |
|---|---|---|
| *E. coli* | DSM 19445 | June 21,2007 |

The strain has been deposited under conditions that assure that access to the culture will be available during the pendency of this patent application to one determined by foreign patent laws to be entitled thereto. The deposit represents a substantially pure culture of the deposited strain. The deposit is available as required by foreign patent laws in countries wherein counterparts of the subject application, or its progeny are filed. However, it should be understood that the availability of a deposit does not constitute a license to practice the subject invention in derogation of patent rights granted by governmental action.

**Claims**

**1.** A method for producing an acidified milk drink, said method comprising:

a) acidifying a milk substrate comprising milk protein; and
b) treating with an enzyme having a protein modifying activity which reduces the isoelectric point of the milk protein;

wherein step b) is performed before, during or after step a).

**2.** The method of claim 1, wherein the acidification comprises fermentation with a lactic acid bacterium.

**3.** The method of claim 1, wherein step a) comprises chemical acidification.

**4.** The method of any of the preceding claims, wherein step b) is performed before or during step a).

**5.** The method of any of the preceding claims, wherein the milk substrate is subjected to pasteurization before step a) and step b) is performed after pasteurization.

**6.** The method of any of the preceding claims, wherein the acidified milk substrate is mixed with a syrup and the mixture is subjected to homogenization.

**7.** The method of any of the preceding claims, wherein the acidified milk drink is in liquid form.

**8.** The method of any of the preceding claims, wherein the acidified milk drink has a viscosity of less than 40 Pa at a shear rate of 300 pr. s.

**9.** The method of any of the preceding claims, wherein the milk substrate has a protein content of more than 5%.

**10.** The method of any of the preceding claims, wherein the acidified milk drink has a milk protein content of less than 3%.

**11.** The method of any of the preceding claims, wherein the milk protein is casein.

**12.** The method of any of the preceding claims, wherein the enzyme has deamidase activity.

**13.** The method of any of the preceding claims, wherein the amino acid sequence of the enzyme has at least 50% identity to SEQ ID: NO 2 or SEQ ID NO: 4.

**14.** An acidified milk drink obtainable by any of the preceding claims.

**15.** An isolated polypeptide having deamidase activity, selected from the group consisting of:

a) a polypeptide having an amino acid sequence which is at least 91% identical to (i) SEQ ID NO: 2, or (ii) amino acids 133-318 thereof;
b) a polypeptide which is encoded by a polynucleotide which is at least 90% identical to (i) SEQ ID NO:1, or (ii) nucleotides 397-954 thereof;
c) a polypeptide which is encoded by a polynucleotide whose complement hybridizes under high stringency conditions with nucleotides 397-954 of SEQ ID NO: 1; and
d) a polypeptide which is encoded by the plasmid contained in *E. coli* DSM 19445.

## Patentansprüche

**1.** Verfahren zur Herstellung eines gesäuerten Milchgetränks, wobei das Verfahren folgendes umfasst:

a) Ansäuerung eines Milchsubstrats, das Milchprotein enthält, und
b) Behandlung mit einem Enzym, das eine Proteinveränderungsaktivität aufweist, die den isoelektrischen Punkt des Milchproteins verringert,

wobei Schritt b) vor, während oder nach Schritt a) durchgeführt wird.

**2.** Verfahren nach Anspruch 1, wobei die Ansäuerung eine Fermentation mit einem Milchsäurebakterium umfasst.

**3.** Verfahren nach Anspruch 1, wobei Schritt a) eine chemische Ansäuerung umfasst.

**4.** Verfahren nach einem der vorherigen Ansprüche, wobei Schritt b) vor oder während Schritt a) durchgeführt wird.

**5.** Verfahren nach einem der vorherigen Ansprüche, wobei das Milchsubstrat vor Schritt a) einer Pasteurisierung unterzogen wird und Schritt b) nach der Pasteurisierung durchgeführt wird.

**6.** Verfahren nach einem der vorherigen Ansprüche, wobei das gesäuerte Milchsubstrat mit einem Sirup vermischt wird und die Mischung einer Homogenisierung unterzogen wird.

**7.** Verfahren nach einem der vorherigen Ansprüche, wobei das gesäuerte Milchgetränk in flüssiger Form ist.

**8.** Verfahren nach einem der vorherigen Ansprüche, wobei das gesäuerte Milchgetränk eine Viskosität von weniger als 40 Pa bei einer Schergeschwindigkeit von 300 pro Sek. aufweist.

**9.** Verfahren nach einem der vorherigen Ansprüche, wobei das Milchsubstrat einen Proteingehalt von mehr als 5 % aufweist.

**10.** Verfahren nach einem der vorherigen Ansprüche, wobei das gesäuerte Milchgetränk einen Milchproteingehalt von weniger als 3 % aufweist.

**11.** Verfahren nach einem der vorherigen Ansprüche, wobei das Milchprotein Kasein ist.

**12.** Verfahren nach einem der vorherigen Ansprüche, wobei das Enzym eine Deamidase-Aktivität aufweist.

**13.** Verfahren nach einem der vorherigen Ansprüche, wobei die Aminosäuresequenz des Enzyms zu mindestens 50 % identisch mit SEQ ID NO. 2 oder SEQ ID NO. 4 ist.

**14.** Gesäuertes Milchgetränk, das durch einen der vorherigen Ansprüche erzeugt werden kann.

**15.** Isoliertes Polypeptid, das eine Deamidase-Aktivität aufweist und aus der folgenden Gruppe ausgewählt wird:

a) Polypeptid mit einer Aminosäuresequenz, die zu mindestens 91 % identisch mit (i) SEQ ID NO. 2 ist oder (ii) Aminosäuren 133-318 hiervon,
b) Polypeptid, das von einem Polynukleotid kodiert ist, das zu mindestens 90 % identisch mit (i) SEQ ID NO. 1 ist oder (ii) Nukleotiden 397-954 hiervon,
c) Polypeptid, das von einem Polynukleotid kodiert ist, dessen Komplement unter hohen Stringenzbedingungen mit Nukleotiden 397-954 von SEQ ID NO. 1 hybridisiert, und
d) Polypeptid, das von dem Plasmid kodiert ist, das in E. *coli* DSM 19445 enthalten ist.

**Revendications**

**1.** Méthode de production d'une boisson lactée acidifiée, ladite méthode comprenant:

a) acidification d'un substrat de lait comprenant de la protéine de lait; et
b) traitement avec une enzyme ayant une activité de modification de protéine qui réduit le point isoélectrique de la protéine de lait;

où la phase b) est effectuée avant, pendant ou après la phase a).

**2.** Méthode selon la revendication 1, où l'acidification comprend la fermentation avec une bactérie d'acide lactique.

**3.** Méthode selon la revendication 1, où la phase a) comprend l'acidification chimique.

**4.** Méthode selon l'une quelconque des revendications précédentes, où la phase b) est effectuée avant ou pendant la phase a).

**5.** Méthode selon l'une quelconque des revendications précédentes, où le substrat de lait est soumis à une pasteurisation avant la phase a) et la phase b) est effectuée après la pasteurisation.

**6.** Méthode selon l'une quelconque des revendications précédentes, où le substrat de lait acidifié est mélangé avec un sirop et le mélange est soumis à une homogénéisation.

**7.** Méthode selon l'une quelconque des revendications précédentes, où la boisson lactée acidifiée est sous forme liquide.

**8.** Méthode selon l'une quelconque des revendications précédentes, où la boisson lactée acidifiée a une viscosité inférieure à 40 Pa à un taux de cisaillement de 300 p. s.

**9.** Méthode selon l'une quelconque des revendications précédentes, où le substrat de lait a une teneur en protéines supérieure à 5%.

**10.** Méthode selon l'une quelconque des revendications précédentes, où la boisson lactée acidulée a une teneur en protéines de lait inférieure à 3%.

**11.** Méthode selon l'une quelconque des revendications précédentes, où la protéine de lait est la caséine.

**12.** Méthode selon l'une quelconque des revendications précédentes, où l'enzyme a activité de déamidase.

**13.** Méthode selon l'une quelconque des revendications précédentes, où la séquence d'aminoacides de l'enzyme a au moins 50% d'identité à SEQ ID: NO 2 ou SEQ ID NO: 4.

**14.** Boisson lactée acidifiée obtenable par l'une quelconque des revendications précédentes.

**15.** Polypeptide isolé ayant activité de déamidase, sélectionné du groupe composé de:

a) un polypeptide ayant une séquence d'aminoacides qui est au moins 91 % identique à (i) SEQ ID NO: 2, ou (ii) les aminoacides 133-318 de celle-ci;
b) un polypeptide qui est codé par un polynucléotide qui est au moins 90% identique à (i) SEQ ID NO:1, ou (ii) les nucléotides 397-954 de celle-ci;
c) un polypeptide qui est codé par un polynucléotide dont le complément s'hybride dans des conditions de stringence élevée avec les nucléotides 397-954 de SEQ ID NO: 1; et
d) un polypeptide qui est codé par le plasmide contenu dans *E. coli* DSM 19445.

Figure 1

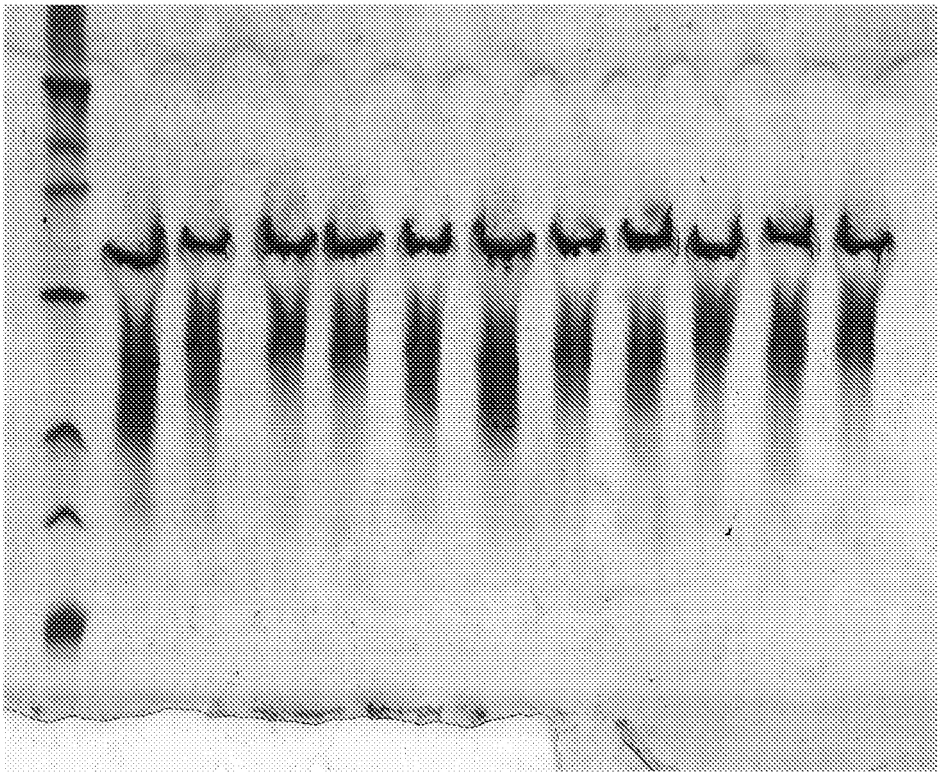

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 976829 A
- US 6756221 B
- WO 2006075772 A
- JP 2003250460 B
- WO 2002068671 A
- WO 9943835 A


### Non-patent literature cited in the description


- **HARTE et al.** *J. Dairy Sci.,* 2003, vol. 86, 1074-1082
- **KIKUCHI, M. ; HAYASHIDA, H. ; NAKANO, E. ; SAKAGUCHI, K.** Peptidoglutaminase. Enzymes for selective deamidation of y-amide of peptide-bound glutamine. *Biochemistry,* 1971, vol. 10, 1222-1229
- **J. SAMBROOK ; E.F. FRITSCH ; T. MANIATIS.** Molecular Cloning, A Laboratory Manual. Cold Spring Harbor, 1989
- **BOLTON ; MCCARTHY.** *Proceedings of the National Academy of Sciences USA,* 1962, vol. 48, 1390
- **RICE, P. ; LONGDEN, I. ; BLEASBY, A.** EMBOSS: The European Molecular Biology Open Software Suite. *Trends in Genetics,* 2000, vol. 16 (6), 276-277, http://emboss.org
- **NEEDLEMAN, S. B. ; WUNSCH, C. D.** *J. Mol. Biol.,* 1970, vol. 48, 443-453
- **WILBUR ; LIPMAN.** *Proceedings of the National Academy of Science USA,* 1983, vol. 80, 726-730
- *Eur J Biochem,* 2001, vol. 268, 1410-1421
- **DIDERICHSEN et al.** A useful cloning vector for Bacillus subtilis. *Plasmid,* 1993, vol. 30, 312